# EUROPEAN PATENT SPECIFICATION

(11) **EP 4 138 961 B1**
(45) Date of publication and mention of the grant of the patent: **05.06.2024**
(21) Application number: 21718137.9
(22) Date of filing: 16.04.2021
(51) Int. Cl.: A61M 5/20, A61M 5/32, A61M 5/315

(54) **ACTIVATABLE DRUG DELVIERY DEVICE WITH SAFETY ASSEMBLY**
AKTIVIERBARE ARZNEIMITTELABGABEVORRICHTUNG MIT SICHERHEITSBAUGRUPPE
DISPOSITIF D'ADMINISTRATION DE MÉDICAMENT AVEC UN MÉCANISME DE SÉCURITÉ

(30) Priority: 23.04.2020 EP 20170998
(43) Date of publication of application: 01.03.2023
(73) Proprietor: Novo Nordisk A/S, 2880 Bagsværd (DK)
(72) Inventor: VAN DER BEEK, Willem, 2880 Bagsværd (DK)
(86) International application number: PCT/EP2021/059889
(87) International publication number: WO 2021/213912

(56) References cited:
- EP-A1- 1 542 744
- EP-B1- 1 542 744
- WO-A1-2012/045350
- WO-A1-2017/144601
- WO-A1-2020/038649
- US-A1- 2018 243 498
- US-B2- 9 242 053

## Description

The present invention relates to a drug delivery device with an inner ring for operating an unlocking mechanism. More specifically the invention relates to a drug delivery device comprising a drive mechanism, an activation mechanism and a turnable activation member for enabling activation of the activation mechanism, in response to a rotation, and for activating the drive mechanism, in response to an axial movement, wherein the inner ring is adapted for rotating the turnable activation member with a reduced risk of unintentional activation of the drive mechanism.

### BACKGROUND OF THE INVENTION

Drug delivery devices for self-administration of different liquid drug formulation presently exist in various shapes and sizes. Some are adapted for connecting to an infusion set, and some are connectable or integrated with an injection needle. The latter type is referred to as injection devices. Some are durable devices comprising a cartridge with a drug reservoir, wherein the cartridge can be changed. Others are disposable devices that are discarded when the cartridge is empty. Disposable devices can be either multi-dose devices, in which the user can set the desired dose size prior to each injection, or single dose devices, capable of administering only a single dose of a given size. The latter exists with so-called "Shield activation", where the cannula is covered by a shield in the front of the device that releases the dose when pressed. The cannula is then exposed only to enter the skin, when the user presses the device against the skin, and thereby depresses the shield, and releases the dose. These injection devices are disposed after a single injection. Alternatively, a dose can be released by a push button at the proximal end or sliding button at the side.

Fixed dose devices are preferable to some users, since they may not feel comfortable with or be capable of operating the device to adjust the correct dose each time. When devices for instance are used by children or older people, simplicity and ease of use is important to avoid user error leading to over- or under dosing. In other cases, the treatment regimen prescribes a fixed dose of e.g. a GLP-1 type of drug.

However, the device itself is responsible for a considerably part of the costs of the unit, not to mention the amount of materials used and thus necessary to dispose. It would therefore be desirable to make a fixed dose device capable of delivering multiple doses of a fixed volume.

An example of a fixed dose device is disclosed in WO2018/007259 filed by Copernicus. The disclosure relates to an injection device for delivering a defined number of equal doses of a fluid substance. The disclosed injection device comprises a housing 1 with an arming mechanism and a dose delivery mechanism arranged along the longitudinal axis of the housing.

An alternative fixed dose device is disclosed in WO2013/034651 filed by Menarini. The disclosure relates to a device for the automatic injection of two doses of a medicament at two successive times. The disclosure describes an automatic injection device comprising a sliding sheath 30 which, when depressed with its front end 3 against the injection site, interacts with cam means 26, 27, 28 to activate the triggering of a plunger 8, controlling the delivery of a drug dose. Devices of same applicant and with a similar functioning are disclosed in WO2013/034647 and WO2011/111006.

WO2020/089167 filed by Novo Nordisk disclose a minimum dose drug delivery device, wherein a push button or ejection button is used to activate a drive mechanism and expel a dose. A dose dial, or dose button, is used to set a minimum dose, whereby a scale drum is moved along a helical path until a lock in the activation mechanism is unlocked. The minimum dose can be a fixed dose obtained, when a maximum dose has been set, i.e., in this case it is only possible to set the maximum dose. The dose button is couple to a reset tube having a first axial position, wherein the reset tube and the dose dial are rotationally coupled, and a second axial position, wherein the reset tube and the dose button is rotationally decoupled. The push button is rotationally coupled to a proximal end of the reset tube, and is biased in the proximal direction by a return spring position between the push button and the dose button. During dose setting the reset tube is in the first axial position, and rotates together with the dose button and the reset tube due to friction. When the minimum dose has been set, the push button is pushed by a finger of the user and moves the reset tube to the second position, which movement activates the drive mechanism. Hereby, the reset tube rotates, but due to friction the push button and rotationally decoupled dose button does not rotate. The user can unlock the activation mechanism by rotating the dose dial. However, alternative mechanism for unlocking the activation mechanism is desirable. The application was published 07.05.2020, which is after the priority date of the present application.

WO2004028598 A1 filed by SHL Medial AB discloses an embodiment of an injection device comprising a mode selector formed as a sleeve surrounding a pen-formed housing. The mode selector is arranged slidably and turnable outside the housing. The inner surface of the mode selector is arranged with two inwardly directed pins. The pins extend through two cut-outs of the main housing, and the cut-outs provide three distinct positions of the pins and thereby the mode selector. Each position corresponds to a specific mode of function, such as LOCKED, UNLOCKED and INJECTION. The positions are preferably marked with suitable indications on the main housing for facilitating the proper use of the device. The pins extend into corresponding recesses in a dose actuating sleeve in order to maneuver the dose actuating sleeve. The injection device further comprises a needle shield axially movable between a retracted state and an extended state. The shield is fixed rotationally to the housing. The rear end of the needle shield comprises two tongues having grooves, with a certain shape to cooperate with protrusions arranged on the dose actuating sleeve. In order to perform an injection the required dose has to be set, which is done by turning the mode selector and moving it axially from the locked position the injection position, whereby the dose actuating sleeve with the protrusions are rotated and moved towards the front end of the device, as shown by dotted lines in Fig. 10D. Hereafter a dose is set by rotating a dose setting knob, and the drive mechanism is activated by moving the shield to the retracted state, whereby the dose selector is returned to the locked position by an axial and rotational movement, as shown in figure 12C. The mechanism between the shield, the dose actuating sleeve and the mode selector ensures that the device can be set in an injection mode before a dose is set and the shield is pushed to the retracted position.

WO 2020/038649 discloses a needle assembly for an injection device, the shield comprises a locked angular position, and an unlocked angular position. The injection device comprises a drive, a dose setting and an activation mechanism for the drive mechanism. The activation mechanism for the drive mechanism comprises a push button and is operated independently from the rotatable shield. WO2017144601 also discloses injection devices with a manually rotatable shield, wherein the shield can be rotated between a locked and an unlocked position. When a dose has been dialled the drive mechanism can be activated. Upon remounting a cap after injection, the cap rotatably engages the shield and is rotated back to the locked position together with the shield.

For injection devices, wherein the activation mechanism is not changeable between a locked and an unlocked position, but where a dose has to be set before the drive mechanism can be activated, as disclosed in WO2004028598, WO2020/038649, WO2017144601, the risk of unintentionally activating the drive mechanism before a dose has been set is not a problem.

WO 2012/045350 discloses an injection device wherein a proximal rotating button has to be rotated from a non-activated position to an activated position before the shield can be moved axially to activate the drive mechanism. The shield is rotationally locked, and the handling of the activating button is independent from the handling of the shield, i.e., the activating button is rotated without rotating the shield.

US9242053 B2 filed by Owen Mumford discloses an injection device with an axially movable shield and a locking dial, which can be moved between a first angular position, wherein the shield is locked, and a second angular position, wherein the shield can be moved axially to activate the drive mechanism. The shield is rotationally locked to the housing through prongs passing slots in a collar mounted to the housing. Therefore, the shield which is the activation member cannot rotate.

US20180243498 filed by Novo Nordisk disclose two embodiments of an injection device which may be triggered by a shield or a proximal push button. In the first embodiment of the disclosure, the injection device comprising a rotatable ring surrounding the shield. The shield is rotationally locked to the housing and axially locked to the rotatable ring. The ring can rotate relative to the shield. The rotatable ring comprises a helical track engaged by a thread segment of the housing, whereby the ring and the shield are moved in an axial direction, when the ring is moved from a first angular position to a second angular position. The ring further comprises an axial track, which is engaged by the thread portion of the housing at the second angular position, whereby the ring and the shield can be moved in the axial direction to allow exposure of the needle and injection. In a second embodiment of the disclosure the shield comprises the helical and the axial track and is rotatably arranged, between an axially locked position, wherein a thread portion of the housing is arranged in the helical track, and an axially movable position, wherein the thread portion of the housing is arranged in the axial track and whereby the drive mechanism can be activated. Both embodiments comprises a dose setting mechanism, so the user has to set a dose and rotate the ring or the shield, before an injection can be performed.

Alternative solutions for minimizing the risk of unintentionally activating the drive mechanism is still needed. Therefore, an unmet need exists for delivering alternative drug delivery devices with an activatable drive mechanism for delivering a dose of medicament, which ensures safe and reliable activation.

Having regard to the above, it is an object of the present invention to provide a user-friendly, safe and robust drug delivery device for delivering a fixed dose of medicament.

### DISCLOSURE OF THE INVENTION

In the disclosure of the present invention, embodiments and aspects will be described which will address one or more of the above objects or which will address objects apparent from the below disclosure as well as from the description of exemplary embodiments.

In a first aspect of the present disclosure is provided a drug delivery device for delivering a dose of a medicament, wherein the drug delivery device comprises:
- a housing assembly comprising an elongate housing structure,
- a drive mechanism for delivering the dose, the drive mechanism being arranged within the elongate housing structure, wherein the drive mechanism is adapted to expel the dose of the medicament, in response to activation,
- an activation mechanism comprising a rotatable tubular activation member adapted to activate the drive mechanism, in response to an axial movement of the activation member, wherein the activation member is adapted to be arranged in: (i) a first angular position, wherein the activation of the drive mechanism is disabled, (ii) a second angular position, wherein the activation member is arranged to be movable in the axial direction, and thereby allows an axial movement enabling activation of the drive mechanism,

wherein the drug delivery device further comprises a ring member surrounding the activation member,
wherein the ring member is connected to the housing assembly, wherein the ring member is arranged to be axially fixed and movable in the rotational direction relative to the housing assembly, wherein the ring member is axially splined to the activation member, whereby the ring member is rotationally locked and movable in the axial direction relative to the activation member,
whereby the ring member is adapted to transfer a rotation to the activation member from the first position to the second position, in response to a rotation of the ring member, whereby a user can rotate the activation member without unintentionally activating the drive mechanism.

Hereby is provided a drug delivery device with an activation mechanism with an activation member operable between a disabled and an enabled position, and an activatable drive mechanism for delivering a medicament, which ensures safe and reliable activation. This is obtained as the ring member minimizes the risk of unintentional activation, when the activation member is operated between the disabled and enabled position.

In a further aspect, the drug delivery device further comprises:
- a medicament reservoir with an outlet at a distal end,
- a movable piston for compression of the reservoir, and
wherein the drive mechanism further comprises a piston rod for moving the piston to compress and expel the medicament through the outlet, an energized power-reservoir operationally arranged for moving the piston rod and for delivering the entire dose.

Hereby, is further provided a power-reservoir for energizing the drive mechanism.

In a further aspect, the drive mechanism comprises a drive tube axially splined to the piston rod, whereby relative axial movement between the drive tube and the piston rod is allowed and relative rotational movement is restricted, wherein the drive tube is adapted to be axially movable relative to the housing assembly between a first axial and non-rotatable position and a second axial and rotatable position, in response to axial movement of the activation member, and wherein the drive tube arranged in the non-rotatable position is rotationally blocked by the housing assembly, and wherein the drive tube arranged in the rotatable position is allowed to rotate and guided by the housing assembly.

Hereby, is provided a drive tube movable drive tube, which can be positioned in an activated position.

In a further aspect, the housing assembly comprises an axial guide portion for blocking rotation of the drive tube in the non-rotatable position, and wherein the housing assembly comprises a helical guide portion for guiding the drive tube in the rotatable position.

In a further aspect, the activation mechanism further comprises a connector for establishing a connection between the activation member and the drive tube, for the activation member being in the second angular position, whereby the connector can transfer an axial movement of the activation member to the drive tube.

Hereby is provided a drug delivery device, which can be configured to have a state wherein the activation member is not in contact with drive mechanism.

In a further aspect, the activation member in the first angular position, is connected to the connector, and the connector is operationally disconnected from the drive tube, whereby activation is disabled, wherein the activation member in the second angular position is connected to the connector, and wherein the connector is operationally connected to the drive tube, whereby activation is enabled.

Hereby is provided a drug delivery device, which can be configured to have a state wherein the activation member is not in contact with drive mechanism, and wherein the connector is operationally coupled to the drive tube, when the activation member is moved from the first to the second angular position.

In a further aspect, the activation member in the first angular position, is operationally disconnected from the drive tube, whereby activation is disabled, wherein the activation member in the second angular position is operationally connected to the drive tube, whereby activation is enabled.

Hereby is provided a drug delivery device, which can be configured to have a state wherein the activation member is not in contact with drive mechanism, and wherein the activation member is operationally coupled to the connector, when the activation member is moved from the first to the second angular position.

In a further aspect, the activation member comprises a blocking structure for blocking against a blocking structure of the housing assembly, wherein the blocking structures are adapted for preventing pure axial movement of the activation member in the first angular position, whereby activation is disabled, wherein the blocking structures are adapted for allowing axial movement of the activation member in the second angular position, whereby activation is enabled.

Hereby is provided a drug delivery device, wherein the activation member is locked against pure axial movement in the first angular position.

In a further aspect, the housing assembly further comprises an inner thread, wherein the piston rod further comprises an outer thread engaging the inner thread of the housing.

In a further aspect, the power reservoir comprises a torsion spring, wherein the drive tube and the torsion spring are adapted for driving the piston rod, in response to activation of the drive tube by the activation member.

Hereby is provided a power reservoir enabling dosing with an almost constant force.

In a further aspect, the activation member is a proximally positioned push button adapted to be moved axially in the distal direction for activation of the drive mechanism.

Hereby, is provided an activation mechanism that can be activated by a thumb.

In a further aspect, the drive mechanism further comprises a pinion and an electric motor adapted to be activated by the activation member, wherein the piston rod further comprises a toothed rack, wherein the toothed rack and the pinion provides a rack-and-pinion mechanism, and wherein the power reservoir comprises a battery, wherein the electric motor, the rack-and-pinion mechanism and the battery are adapted for driving the piston, in response to activation of the electric motor by the activation member.

Hereby is provided an electrically driven drive mechanism.

In a further aspect, the housing comprises a releasable locking structure for holding the piston rod, and wherein the activation mechanism is adapted to release the piston rod from the releasable lock of the housing, wherein the power reservoir comprises a compression spring for driving the piston rod, in response to activation by the activation member.

Hereby, is provided drive mechanism with a compression spring directly providing an axial force.

In a further aspect, the drug delivery device is an injection device comprising a needle cannula.

In a further aspect, the drug delivery device is a fixed dose injection device, adapted for delivering a predefined fixed volume of medicament.

In a further aspect, the drug delivery device is adapted to be a multi-use fixed dose drug delivery device, wherein the activation member is adapted to be operated a plurality of times and thereby moved: (i) from the first angular position to the second angular position to enable activation, (ii) in the axial direction to activate the drive mechanism, (iii) from the second angular position to the first angular position to disable activation.

In a further aspect, the housing comprises a helical guide for guiding the activation member in the axial direction and into the housing, in response to rotating the activation member from the first angular position to the second angular portion.

In another aspect, is provided a safety assembly for a drug delivery device for delivering a dose of a medicament, wherein the drug delivery device comprises:
- a housing assembly comprising an elongate housing structure,
- a drive mechanism for delivering the dose, the drive mechanism being arranged within the elongate housing structure, wherein the drive mechanism is adapted to expel the dose of the medicament, in response to activation,
- an activation mechanism comprising a rotatable tubular activation member adapted to activate the drive mechanism, in response to an axial movement of the activation member, wherein the activation member is adapted to be arranged in: a first angular position, wherein the activation of the drive mechanism is disabled, a second angular position, wherein the activation member is arranged to be movable in the axial direction, and thereby allows an axial movement enabling activation of the drive mechanism,

wherein the safety assembly comprises the activation member, a housing insert portion and a ring member surrounding the activation member, wherein the housing insert portion is adapted to be fixed to the housing assembly,
wherein the ring member is connected to the housing insert portion, wherein the ring member is arranged to be axially fixed and movable in the rotational direction relative to the housing insert portion, wherein the ring member is axially splined to the activation member, whereby the ring member is rotationally locked and movable in the axial direction relative to the activation member,
whereby the ring member is adapted to transfer a rotation to the activation member from the first position to the second position, in response to a rotation of the ring member, whereby a user can rotate the activation member without unintentionally activating the drive mechanism.

In another aspect is provided a method of using a drug delivery device or a safety assembly according to the above description, wherein the method comprises:
- turning the ring member from the first angular position to the second angular position and thereby enabling activation of the drive mechanism, without directly touching the activation member, and thereby not expelling a dose during rotation of the activation member.

### BRIEF DESCRIPTION OF THE DRAWINGS

In the following embodiments of the invention will be described with reference to the drawings:
Figure 1 illustrates an exploded view of a fixed dose drug delivery device with multiple doses according to a first embodiment of the present disclosure.
Figure 2 illustrates operation of the drug delivery device of figure 1.
Figures 3A-3B illustrate details of the drug delivery device of figure 1 in different operational states.
Figure 4A illustrates an exploded view of a fixed dose drug delivery device with multiple doses according to a second embodiment of the present disclosure.
Figure 4B illustrates the drug delivery device of figure 4A in a cross-sectional view.
Figures 5A1-5C2 illustrate details of the drug delivery device of figure 4A in different operational states. Figures 5A1-5A4 relate to the same state, figure 5B1-5B2 relate to the same state and figure 5C1-5C2 relate to the same state.
Figure 6 illustrates an exploded view of an embodiment of safety assembly for implementation in an drug delivery device.
Figure 7 shows a profile view of the safety assembly of figure 6 in an assembled out-of-pack state, wherein the shield is in its distalmost position.
Figure 8A shows a perspective view of the safety assembly of figure 7 in an initiated state, wherein the shield is in a position disabling for activation.
Figure 8B shows a perspective view of the safety assembly of figure 8A, wherein the shield is turned to a position between disabled and enabled.
Figure 8C shows a profile view of the safety assembly of figure 8B, in the shield unlocked state, wherein the shield is turned to a position enabling activation.
Figure 9A-9C illustrate details of structures of the safety assembly.

In the figures like structures are mainly identified by like reference numerals. Reference numbers followed by the letter "a" is used to denote the distal end of the structure, and numbers followed by "b" is used to denote the proximal end. Reference numbers comprising a first number followed by a "." and a second number is used to denote a functional or structural detail of a structure. In this way the first number indicates a primary (relatively large) structure and the second number indicates a secondary (relatively small) structure or a specific function. Reference numbers followed by the letters c, d and e indicate features with rotational symmetry.

### DESCRIPTION OF EXEMPLARY EMBODIMENTS

When in the following terms such as "upper" and "lower", "right" and "left", "horizontal" and "vertical" or similar relative expressions are used, these only refer to the appended figures and not necessarily to an actual situation of use. The shown figures are schematic representations for which reason the configuration of the different structures as well as their relative dimensions are intended to serve illustrative purposes only. When the term member is used for a given component it can be used to define a unitary component or a portion of a component, having one or more functions.

In the following detailed description, numerous specific details are set forth in order to provide a thorough understanding of the present disclosure. However, it will be apparent to one of ordinary skill in the art that the present disclosure may be practiced without these specific details.

It will also be understood that, although the terms first, second, etc. may be used herein to describe various elements or positions, these elements or positions should not be limited by these terms. These terms are only used to distinguish one element or position from another. For example, a first subject could be termed a second subject, and, similarly, a second subject could be termed a first subject, without departing from the scope of the present disclosure. The first subject and the second subject are both subjects, but they are not the same subject. Furthermore, the terms "subject," "user," and "patient" are used interchangeably herein.

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

As used herein, the term distal and proximal end is in analogy with the terminology from anatomy used to describe the end situated away from or nearest the point of attachment to the body. Therefore, the distal end of an injection device is defined in a context, where a user holds the device in a ready to inject position, whereby the end with the injection needle will be the distal end and the opposite end will be the proximal end. Furthermore, distal and proximal ends of individual components of the device is also defined in that context.

As used herein, rotational symmetry, is a property of a structure when it appears the same or possess the same functionality after some rotation by a partial turn. A structure's degree of rotational symmetry is the number of distinct orientations in which it appears the same for each rotation. Rotational symmetry of order n, wherein n is 2 or more, is also called n-fold rotational symmetry, or discrete rotational symmetry of the n^{th} order, with respect to a particular point (in 2D) or axis (in 3D), which means that rotation by an angle of 360°/n does not change the object. The property of the structure may both relate to the visible appearance and the functional capability of structural feature.

As used herein, the term clockwise direction is used to describe the direction that the hands of a clock rotate as viewed from in front. Therefore, the clockwise rotation of the injection device is the clockwise rotation observed, when viewing the device from the distal face, i.e., when viewed in the proximal direction. Counterclockwise or anticlockwise rotation is defined as the opposite direction.

As used herein, the proximal face is the face of the device as viewed from the proximal end and in the distal direction, wherein a distal face is the face of the device as viewed from the distal end and in the proximal direction.

As used herein, a positive axial or longitudinal direction is defined from the proximal end towards the distal end. A positive axial direction and a distal direction are used interchangeably with the same meaning. Similar, the definitions of a negative axial direction and a proximal direction are used interchangeably with the same meaning. A central axis of the device is defined through the centre of the injection device in the positive axial direction, which is also referred to as a longitudinal axis, with the same meaning.

As used herein, a positive radial direction is defined along a radial axis originating at the central axis and with a direction perpendicular to the central axis.

A positive circumferential or positive angular direction is defined for a point positioned at a radial distance from the central axis, wherein the positive circumferential direction is the counterclockwise direction when observed in the negative axial direction. The circumferential direction is perpendicular to the axial and radial direction.

Both the radial and the circumferential direction are herein referred to as transverse directions, as they are transverse or normal to the axial direction. The transverse plane is herein defined as a plane spanned by two vectors in the radial and circumferential direction, and with the central axis as the normal vector.

As used herein, axial movement of a structure is used to describe a movement, wherein the displacement vector of the structure has a component in the axial direction. A translational movement is used to describe a uniform motion in the axial direction only. A pure, strict or uniform axial movement is the same as a translational movement and the terms are used interchangeably.

Radial movement of a structure is used to describe a movement, wherein the displacement vector of the structure has a component in the radial direction. A pure or strict radial movement is used to describe a uniform motion in the radial direction only. Thus a pure, strict and uniform radial movement is the same and the terms are used interchangeably.

Circumferential or rotational movement of a structure is used to describe a movement, wherein the displacement vector of the structure has a component in the circumferential direction. A pure or strict circumferential movement is used to describe a uniform motion in the circumferential direction only. Thus a pure, strict and uniform circumferential movement is the same as pure, strict and uniform rotational movement, and these terms are used interchangeably. The definition of rotational movement for a structure also encompasses the special case, wherein the structure comprises a central axis defining the axis of rotation. In this special case, all the positions of the structure, which are off the central axis, are subject to a circular circumferential movement, whereas the displacement vector of the positions on the central axis is zero. Therefore, a structure rotating about its own central axis is said to perform a rotational movement.

A helical movement of a structure is used to describe a combined axial and rotational movement, wherein the displacement vector of the structure comprises a circumferential and an axial component. The definition of helical movement for a structure also encompasses the special case, wherein the structure comprises a central axis defining an axis of rotation. In this special case, all the positions of the structure, which are off the central axis, are subject to a helical movement, whereas the displacement vector of the positions on the central axis only comprises an axial component. Therefore, a structure rotating about its own central axis and moving in an axial direction is said to perform a helical movement.

In this context pure, strict and uniform movements are abstract mathematical definitions, and these terms are used to describe an ideal or abstract movement of the structures. Therefore, a structure in a real device should not be expected to exhibit this ideal behaviour, rather such a structure should be expected to move in a pattern approximating such an ideal movement.

As used herein a right-handed thread or helical portion is a thread or helix portion whose helix moves in the positive axial direction, when a screw with the thread is turned counterclockwise.

A screw with a right handed-thread is by convention the default thread, and is screwed in the positive direction by counterclockwise rotation usually performed by the right hand. Similar, a screw with a left-handed thread is screwed in the positive direction by a clockwise rotation, and can thus be performed with the left hand and mirror the movement of the right hand operating a right-handed thread.

The present disclosure describes a drug delivery device for delivering a dose of a medicament, wherein the drug delivery device comprises a housing assembly, a drive mechanism, and an activation mechanism. The drug delivery device further comprises an elongate housing structure and a safety assembly.

The drive mechanism is adapted for delivering the medicament dose, and is arranged within the elongate housing structure. The drive mechanism is adapted to expel the dose, in response to activation.

The activation mechanism comprises a rotatable tubular activation member which may be in the form of a shield or a push button adapted to activate the drive mechanism, in response to an axial movement of the activation member. The activation member is further adapted to be arranged in a first angular and a second angular position. In the first angular position, the activation of the drive mechanism is disabled, i.e., the activation member is either locked by the housing, disconnected from the drive mechanism or both.

In the second angular position, the activation member is arranged to be movable in the axial direction, and thereby allows an axial movement enabling activation of the drive mechanism. The activation member is locked, disconnected or both in the first position, and the activation member is unlocked, connected or both in the second position.

The safety assembly comprises a circular ring member, member surrounding the activation member. The axial extension of the ring member can have a tubular shape and can be either shorter or longer than the diameter.

The ring member is connected to the housing assembly, and is arranged to be axially fixed to the housing assembly, while movement in the rotational direction relative to the housing assembly is enabled or allowed. The ring member is axially splined to the activation member, i.e., the activation member can have an axially extending rib fitting into an axially extending groove of the ring member. Thereby, the ring member is rotationally locked activation member, while the ring member it still movable in the axial direction relative to the activation member.

Thereby, the ring member is adapted to transfer a rotation to the activation member from the first position to the second position, in response to a rotation of the ring member. Hereby, a user can rotate the activation member without unintentionally activating the drive mechanism.

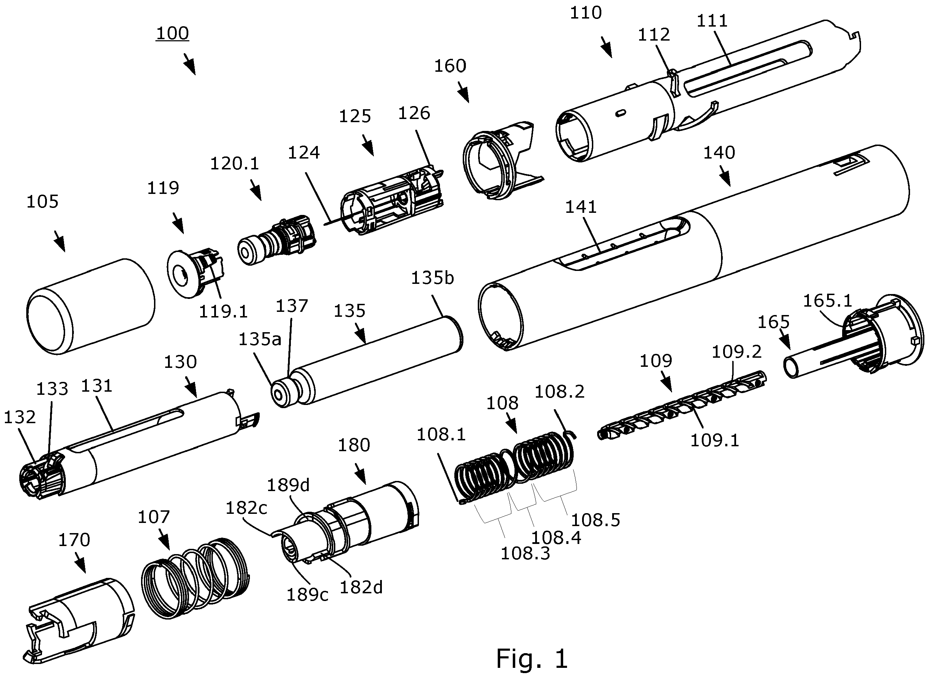

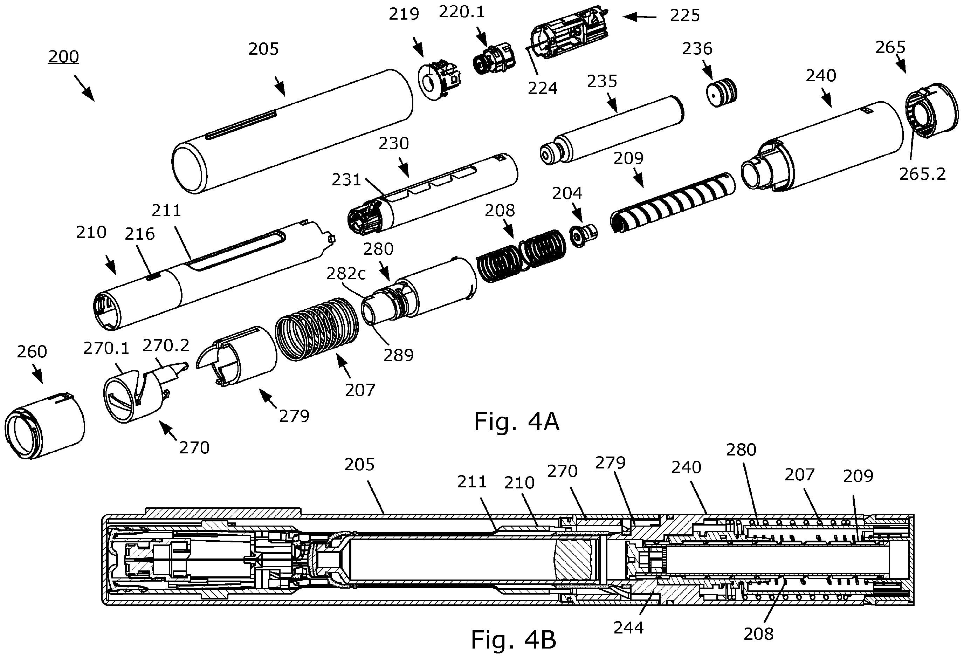

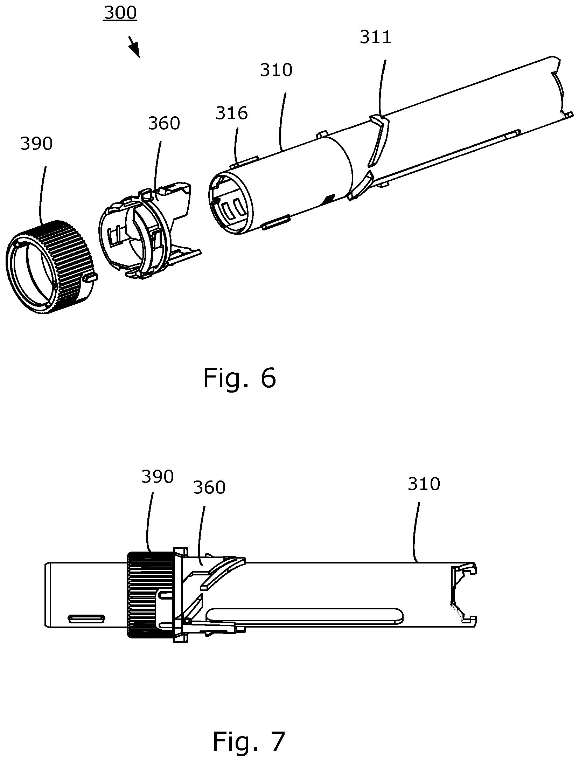

Figs. 1-3 illustrate a first embodiment of a drug delivery device 100 for delivering a plurality of fixed doses of a medicament. Activation of the drive mechanism is disabled, by positioning a shield 110 at a first angular position. Activation of the drive mechanism is enabled, by positioning the shield 110 in a second angular position. Figs. 4-5 illustrate a second embodiment 200 of a multi-use fixed dose drug delivery device. Both embodiments may be modified to cooperate with a safety assembly according to the present disclosure. Figs. 6-8 illustrate an embodiment of a safety assembly 300 with the ring member 390. The drug delivery devices 100 and 200 can both be modified to cooperate with the safety assembly 300.

European patent applications 19217357.3, 19217323.5, 19217333.4, 19217339.1, 19217358.1, 19217343.3 and 19217331.8 discloses further technical details and mechanism of the first and second drug delivery device, which is not described in the present disclosure.

### First embodiment of a fixed dose drug delivery device for multiple injections

Figure 1 illustrates an exploded view of the drug delivery device 100. Figure 1 illustrates a cap 105, a shield tip 119, a shield following portion 120.1 of a cleaning module, a needle hub 125 with a needle cannula 124, a housing insert portion 160, a tubular elongate needle shield structure 110, a cartridge holder 130, a cartridge 135, a tubular elongate housing structure 140, a connector 170, a shield return spring 107, a drive tube 180, a dose drive spring 108, a piston rod 109, and a spring base 165.

### Housing assembly

The drug delivery device comprises a housing assembly, providing a rigid frame with guides and connectors for guiding and connecting the other components of the device. The housing assembly comprises the housing insert portion 160, the tubular elongate housing structure 140, the cartridge holder 130 and the spring base 165. After final assembly these structures are fixedly connected. The elongate housing structure 140 comprises an internal thread for engaging an outer thread of the piston rod. The housing insert portion 160 comprises a cap snap at the end of a track for a bayonet coupling with the cap. The housing insert portion 160 further comprises a proximal edge for guiding the shield. The housing assembly may be referred to as the housing.

### Needle shield assembly

The drug delivery device further comprises a needle shield assembly comprising the shield tip 119 and the elongate shield structure 110. The elongate shield structure 110 comprises a window 111 for inspection of the drug, the elongate shield can be arranged in a first position of overlapping with the cartridge holder window 131, and in a second position with no overlap, wherein a solid portion of the elongate shield structure covers the window 131 in the second position. In this exemplified embodiment, the elongate shield structure 110 provides the activation member, which will be described in further details in the following. The needle shield assembly may be referred to as the needle shield. The elongate shield structure further comprises a step-wise helical guide structure 112, for turning rotational movement into axial movement, i.e., the step-wise helical guide is adapted to guide a helical movement of the shield in cooperation with structures or guides on the inner surface of the housing assembly.

### Cartridge holder

The cartridge holder 130 is adapted for receiving the cartridge 135. The cartridge holder comprises a window 131 for inspecting the drug in the cartridge 135. The cartridge holder 130 comprises a flexible arm for snapping to a neck portion 137 of the cartridge.

### Cartridge

As further shown in figure 1A, the elongate cartridge 135 comprises a distal end 135a sealed by a pierceable septum and an open proximal end 135 closed by a piston. The piston is not shown on figure 1. The cartridge comprises a reservoir containing the plurality of fixed doses of a medicament. At the distal end 135a is provided a septum capped on by a cap. The cap and a main portion of the reservoir is separated by the neck portion 137.

### Needle assembly

The drug delivery device further comprises a needle assembly comprising a needle hub 125 and a reusable needle cannula 124. The cannula comprises a proximal end for piercing the pierceable septum and for establishing fluid communication with the reservoir, and a distal end for insertion into the skin of a subject or user of the device.

### Piston washer

A piston washer, although not shown on figure 1, can be connected to the piston rod to provide a pressure foot for contacting the piston. Alternatively, a dose measuring module for measuring the relative rotation between the piston rod and the piston can be provided between the piston rod and the piston. Such a measuring module also provides a suitable pressure foot. Such a dose measuring module is described in WO 20141128155, titled "Dose capturing cartridge module for drug delivery device. Alternatively, the piston rod directly contacts the piston".

### Cap

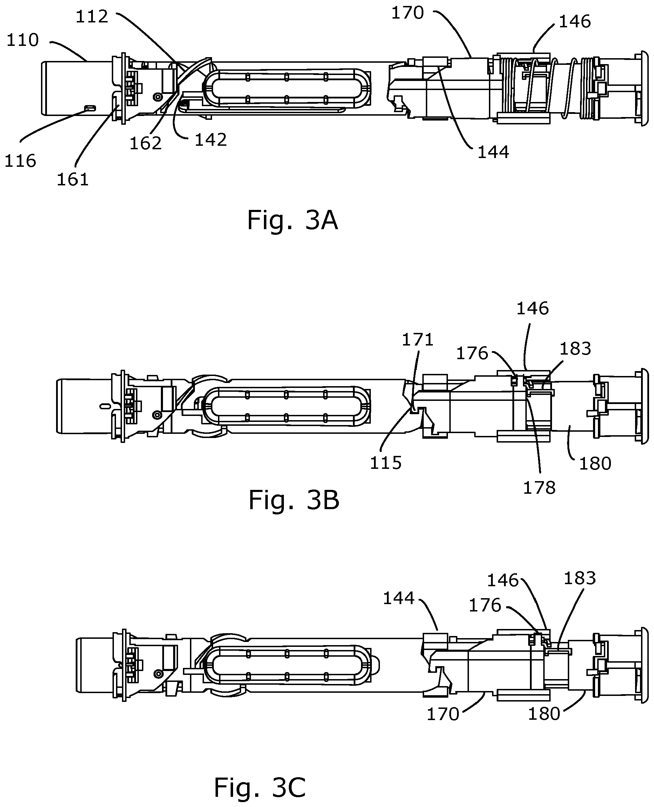

The cap 105 is adapted for releasable mounting to the housing insert portion 160. The cap comprises an inner surface with a protrusion adapted to be guided by the axial and the circumferential cap mount track 161 (Fig. 3A). The protrusion is further adapted to cooperate with a snap lock provided in the circumferential track 161, and thereby releasably lock the cap 105 to the inner housing portion 160. The cap is adapted to be mounted and demounted by a sequential axial and rotational movement, and thereby provides a bayonet coupling together with the drug delivery device. The inner surface of the cap 105 further comprises an axially extending rib (not shown) protruding from the inner surface and adapted for transferring a torque, during initialization, to the shield structure 110 through an axially extending rib 116 (Fig. 3A).

### Spring base

The spring base 165 is fixedly mounted to the housing structure 140 at the proximal end and is adapted to receive and support a compressible torsional drive spring 108.

### Drive spring and drive tube

The drive spring 108 is pre-strained or winded up and positioned between the spring base 165 and the drive tube 180. The drive spring is further adapted to induce a torque on the drive tube 180, whereby the medicament can be expelled. The drive spring comprises torsional sections 108.3, 108.5, wherein the spacing between the coils is relatively small and a compressible section 108.4 adapted to transfer an axial force to the drive tube after compression and during expelling of the medicament. The ability to drive the drive tube in an axial direction enables an end of dose mechanism, and to enable a resetting of the drive tube. The drive tube comprises an axial portion 182, providing a rotational stop in a non-rotational position, and a helical portion 189 for guiding a helical movement in a rotational position.

### Return spring

The connector return spring 107 is positioned between the spring base 165 and the connector 170 and is adapted to urge the connector in the distal direction.

### Cleaning assembly

Cleaning the needle between injections allows the same integrated needle to be used a plurality of times in a clean condition. Therefore, in an alternative embodiment of the present disclosure, the drug delivery device comprises a cleaning assembly. The movable shield structure 110 is fixedly connected to the cleaning assembly through the shield following portion 120.1, and the principles of the cleaning module is disclosed in further details in WO2019/101670. The cleaning assembly comprises a cleaning agent, which keeps the distal end of the needle cannula 124 clean between injections. The shield following portion 120.1 of the cleaning module is fixedly connected to the shield tip 119, which again is fixedly connected to the movably arranged elongate needle shield structure 110. The shield tip 119 can be click fitted to the needle shield structure 110 via resilient arms 119.1 engaging the elongate shield structure 110, such that the cleaning chamber assembly 120 follows axial and rotational movements of the movably arranged shield structure 110. The shield structure 110 which is connected to the cleaning assembly 120 is movably arranged relative to the needle cannula 124, which is fixed to the housing.

The cleaning assembly 120 preferably contains a chamber with a liquid cleaning agent which in one example can be the same preservative as contained in the liquid drug in the cartridge 135. In a preferred example, the cleaning agent is the identical same preservative containing pharmaceutical liquid drug as contained in the cartridge 135, which is filled into the chamber of the cleaning module during the initiation of the drug delivery device. In an alternative embodiment the cleaning agent is embedded in a porous plug. In a further alternative the cleaning agent is embedded in a matrix of a solid plug.

The shield can be arranged in different positions. An initial position defined by an initial angular position and a corresponding initial axial position. A locked position defined by a locked angular position and a corresponding locked axial position. An unlocked distal position defined by an unlocked angular position and a corresponding distal unlocked axial position. The movable shield can be changed by a combined rotational and proximal movement from the initial position to the locked position, wherein the shield is axially locked. In both positions the needle tip is covered by the shield and contained in the cleaning chamber assembly. During use the shield can be further rotated and moved further in the proximal direction in a helical movement to the unlocked distal position, whereby the tip is uncovered. By moving the shield further in the proximal direction in an axial movement the shield uncovers a larger portion of the needle and an injection can be made. After injection the shield is moved back to the locked position, whereby the needle tip is cleaned.

If it for some reason should be desired to reuse the needle without cleaning the needle, the cleaning module can be left out.

### Operation of the device

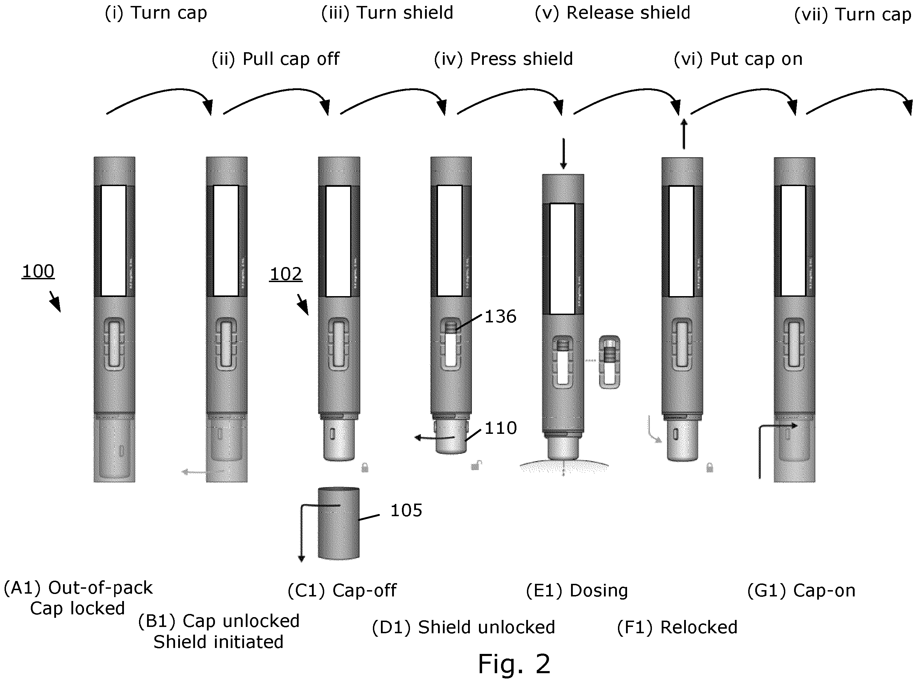

Figure 2 is used to describe the working principles of an embodiment of the disclosure from a user perspective, and a more detailed description of the working principle will be described later with reference to figures 3A-3C. Figure 2 shows the user operations of the drug delivery device 100 for taking a first dose. The drug delivery device can be stored and delivered in a secondary packaging, and in the out-of-pack state (A1) the drug delivery device has been unpacked from the secondary packaging.

As illustrated in figure 2, when the user desires to take a first fixed dose, the drug delivery device is unpacked and thereby provided in the out-of-pack state (A1). Thereafter the drug delivery device is initiated by the user. Initiation can be done by grapping a main portion 102 of the device with the right hand and the cap 105 with the left hand. Hereafter, the user turns the cap in the counterclockwise direction (for the illustrated example). Hereby, the cap snaps off a cap snap of the housing assembly and engages the needle shield, whereby the needle shield follows the rotation of the cap 105 until the cap 105 has been turned to a rotational stop. Due to the step-wise helical guide 112 of the shield, the needle shield is subject to a combined proximal and rotational movement, in response to the user turning the cap. Furthermore, by this initial rotation of the cap and the combined rotational and proximal movement of the needle shield, the needle cannula 124 pierces the septum of the cartridge 135, and thereby establishes fluid communication with a drug reservoir in the cartridge 135. Furthermore, in this operation the cartridge 135 is proximally displaced and pushed against the piston rod 109 or the piston washer 104. As the cannula has established fluid connection, and as the piston is arranged in abutment with the piston rod, the integrated needle is primed. As the cap reaches the rotational stop, the drug delivery device is positioned in the cap unlocked and initiated state (B1), wherein the cap is unlocked and positioned to be taken off.

In the following step the user pulls the cap 105 of, whereby the drug delivery device is arranged in the cap-off state (C1), and wherein the shield is locked against axial translation.

Hereafter, the user manually turns the shield in the counterclockwise direction, whereby the device is arranged in a shield unlocked state (D1), the shield is arranged in an unlocked position and can be pressed proximally into the housing. Due to the step-wise helical guide 112 of the shield, the shield is subject to a combined proximal and rotational movement when operated between the cap-of state and the shield unlocked state. During the rotational unlocking movement of the needle shield, the needle shield uncovers the cartridge inspection window 141 in the housing, whereby the drug in the cartridge can be inspected. In addition, the piston 136 is also visible in the inspection window 141, and the position of the piston 136 relative to a fixed dose scale on the housing indicates the progression of the piston during use, and thereby indicates the remaining number of fixed doses in the reservoir. In figure 2, in the shield unlocked state (D1), the piston 136 is arranged in the initial position and 4 doses are remaining in the reservoir. During the proximal movement of the needle shield, the distal end of the needle tip protrudes through the septum at the distal end of the cleaning assembly 120, whereby any excess pressure in the needle is released.

Hereafter, the user presses the needle shield against the injection site, whereby the shield and the connector 170 is proximally displaced against the force of the shield return spring 107. Hereby, the needle is inserted into the skin or subcutaneous layer of a patient. By this operation, the axial movement of the shield triggers the drive mechanism, and a fixed dose is delivered through the needle cannula in a dosing state (E1). At the end of dose, the piston 136 has moved to the next position, which is indicated by the fixed dose residual scale on the housing, and the drug delivery device can be removed from the injection site. The cut-out window of the residual scale shows the piston in the next position. As the piston 136 progresses under the dosing state it can be useful to define substates for a respective dosing state: an initial dosing state (E2.1) and a final dosing state (E2.2), wherein the piston is in a pressurized proximal position and a relaxes distal position, respectively.

After the dose has been completed, the user removes the device from the skin, and the pressure is thereby released from the shield. Consequently, the shield moves in the distal direction due to the action of the return spring 107. Due to step-wise helical guide 112 the shield, the shield is subject to a distal movement followed by a combined distal and rotational movement, whereby the shield automatically returns to a relocked state (F1).

Hereafter, the user puts on the cap 105 by an axial movement to put the device in a capon state (G1), which is the last state shown in the sequence shown in figure 2. The cap unlocked state and the cap on state, within the same sequence, differs technically in that the cartridge comprises a dose less in the cap on state. Finally, the cap is turned, and thereby snap locked to the housing assembly.

A subsequent dose can be administrated in a similar manner, but without requiring initialization. When the last dose has been administered, it is not possible to activate the drive mechanism again.

Figure 3A to 3C illustrate further details of the operation in specific states. Figure 3A illustrates the drug delivery device 100 in the cap-off state (C1), wherein the drug delivery device has been initialized, the shield is axially locked (i.e., activation disabled) and the cap is taken off. Figure 3B illustrates the drug delivery device in the shield unlocked state (D1), wherein the shield 110 is axially unlocked (i.e., activation enabled). Figure 3C illustrates the drug delivery device 100 in the dosing state (E1), wherein a dose is expelled.

As illustrated on figure 3A the housing insert portion 160 comprises a proximal guide 162 cooperating with the step-wise helical guide 112 of the shield. As also illustrated, the housing structure comprises on an inner surface a distal guide 142 for cooperating with the step-wise helical guide 112, a middle guide 144 and a proximal guide 146 for cooperating with the connector 170. Figure 3A illustrates that the shield 110 is in contact with the connector 170.

Figure 3B illustrate that after rotation of the shield 110, the shield 110 has been moved in the proximal direction and has engaged or hooked the connector 170. In this position, an activation tab 178 on an inner surface of the connector 170 is brought in axial abutment with a tab 183 on an outer surface of the drive tube 180. A connector arm 176 of the connector 170, can in this state be axially guided in a proximal direction along the proximal guide 146 of the housing. Hereby, the connector 170 can push the drive tube in the proximal direction. The drive tube 180 is positioned in a rotationally locked state, wherein the axially extending guide portion 182 is rotationally blocked against a corresponding axial guide portion of the housing (not illustrated). The drive tube 180 can thereby be guided in an axial proximal direction without rotation.

Figure 3C illustrate the connector has been moved in a proximal position, when compared to the position of figure 3B. Hereby, the drive tube 180 has been moved to a proximal position, wherein the torsional drive spring 108 has been compressed, and wherein the axial guide portion 182 has disengaged the housing, and rotation is therefore allowed. In this rotational position, and due to the torque provided by the torsional drive spring 108, the drive tube will start to rotate. Due to the helical guide 189 and the distal force from the compressed drive spring 108, the drive tube will be guided distally in a combined distal and rotational movement, i.e., a helical movement.

### Second embodiment of a fixed dose drug delivery device for multiple injections

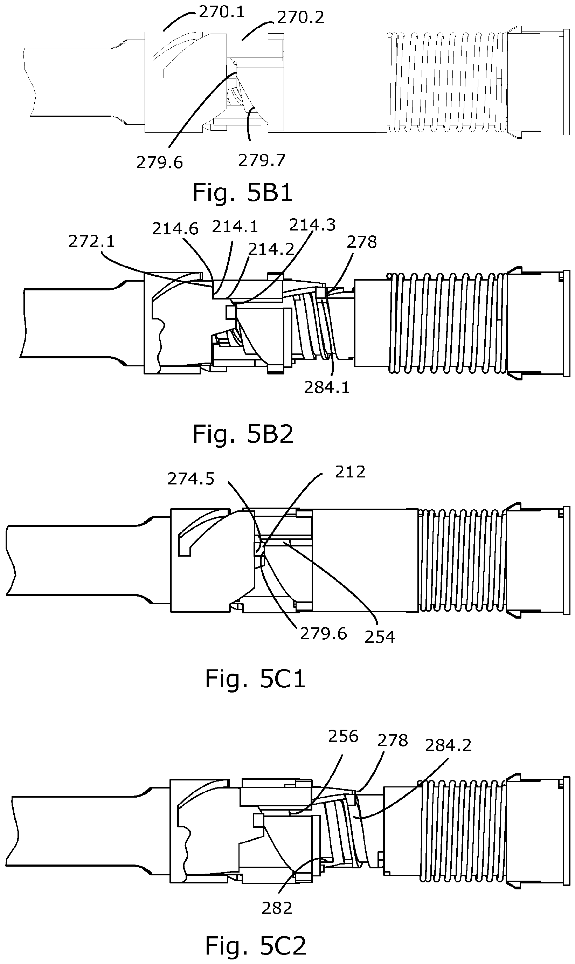

Figs. 4-5 illustrate a second embodiment of a multi-use fixed dose drug delivery device 200. Figure 4A shows an exploded view of the drug delivery device, and figure 4B shows a cross sectional view. Figures 5B1-5C2 illustrate further details of the operation in specific states.

Figure 4A shows a cap 205, a shield tip 219, a shield following portion 220.1 of a cleaning module also comprising a movable portion, corresponding to the movable portion 120.2 in the first embodiment 1. Figure 17 further shows a needle hub 225 with a needle cannula 224, a tubular elongate housing structure 240, and a housing cap portion 260 to be connected at a distal end of the housing structure 240. The figure further shows a tubular elongate needle shield structure 210, a cartridge holder 230, a cartridge 235, a connector 270, a relock tube 279, a shield return spring 207, a drive tube 280, a dose drive spring 208, a piston rod 209, and a spring base 265. The figure further shows a piston washer 204 comprising a ratchet arm and an outer thread for engaging a toothed ring and an inner thread at the inner surface of the piston rod 209, respectively, whereby a zero point adjustment with respect to the piston in the cartridge 235 can be performed. Figure 4B illustrates a cross sectional view of the drug delivery device 200, wherein the drug delivery device is in an out-of-pack state. The cross-sectional plane cuts through the windows 211 of the shield.

### Housing assembly

The drug delivery device comprises a housing assembly, providing a rigid frame with guides and connectors for guiding and connecting the other components of the device. The housing assembly comprises the housing cap portion 260, the tubular elongate housing structure 240, the cartridge holder 230 with a window 231 and the spring base 265. After final assembly these structures are fixedly connected, and the housing assembly can provide a frame of reference for describing the relative movement and position of the other structures. The elongate housing structure 140 comprises an internal thread for engaging an outer thread of the piston rod.

The drug delivery device 200 comprises a drive mechanism and a triggering or activation mechanism. The drive mechanism comprises the piston rod 209, the drive spring 208, and the drive tube 280, and for expelling a dose the structures are operationally arranged in the housing. The triggering mechanism comprises the elongate shield structure 210 and the connector 280, and for triggering the dose expelling mechanism the structures are operationally arranged in the housing.

### Needle shield assembly

The drug delivery device further comprises a needle shield assembly comprising the shield tip 219 and the elongate shield structure 210. The elongate shield structure 110 comprises a window 211 for inspection of the drug, the elongate shield can be arranged in a first position of overlapping with the cartridge holder window 231, and in a second position with no overlap, wherein a solid portion of the elongate shield structure covers the window 231 in the second position.

### Cartridge and cartridge holder

The cartridge holder 230 is adapted for receiving the cartridge 235. The cartridge holder comprises the window 231 for inspecting the drug in the cartridge 235. The cartridge 135 and the cartridge holder 230 is structurally and functionally similar to the cartridge 135 and cartridge holder 130 of the first embodiment, respectively.

### Needle assembly

The needle assembly comprising needle hub 225 and needle 224 are structurally and functionally similar to the needle assembly of the first embodiment.

### Cap

The cap 205 is adapted for releasable mounting to the housing cap portion 260. The cap comprises an inner surface with a protrusion adapted to couple with a bayonet coupling track. The inner surface of the cap 205 further comprises an axially extending rib (not shown) protruding from the inner surface and adapted for transferring a torque to the shield structure 210 through an axially extending rib 216 on the outer surface of the shield. The cap 205 is structurally and functionally similar to the cap 105 of the first embodiment, with an exception that the cap 205 also encloses a main portion of the shield and the cartridge.

### Spring base

The spring base 265 is fixedly mounted to the housing structure 240 at the proximal end and is adapted to receive and support a compressible torsional drive spring 108.

### Drive spring

The drive spring 208 is pre-strained or winded up and positioned between the spring base 265 and the drive tube 280. The drive spring is further adapted to induce a torque on the drive tube, whereby the medicament can be expelled. The drive spring comprises torsional and compressible sections. The ability to drive the drive tube in an axial direction enables an end of dose mechanism, and to enable a resetting of the drive tube.

### Return spring

The connector return spring 207 is positioned between the spring base 265 and the re-lock tube 279 and is adapted to urge the relock tube in the distal direction. In a return arrangement, the relock tube 279 abuts the shield 210, and the shield engages the connector, whereby the shield 210 and the connector 270 can be returned together with the rolock tube 279.

### Cleaning assembly

The cleaning assembly is structurally and functionally similar to the cleaning assembly of the first embodiment.

### Operation of the device

The drug delivery device 200 is operated in a similar manner as illustration for the drug delivery device 100 in figure 2.

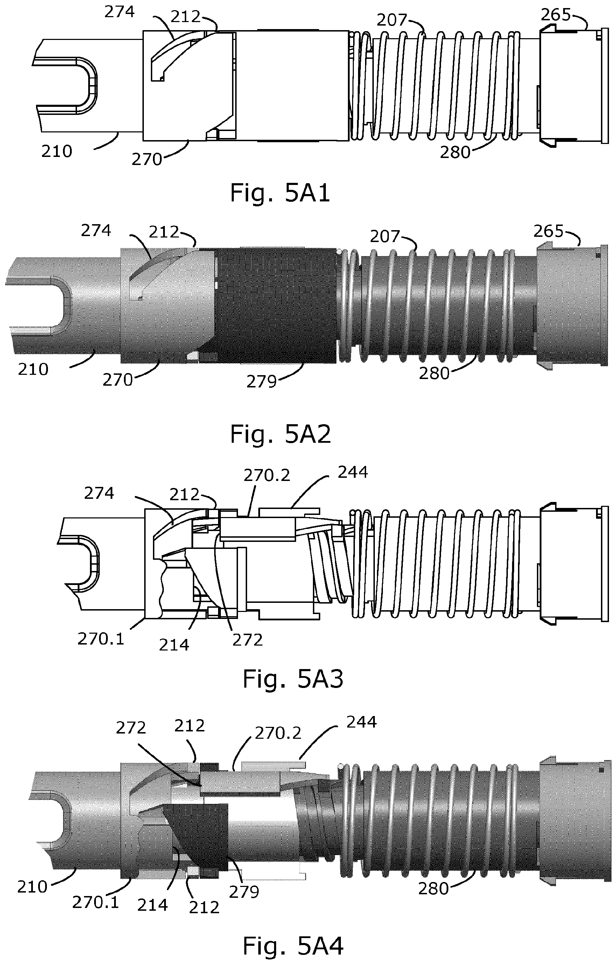

Figures 5A1-5A4 illustrate the drug delivery device 200 in a cap-off state, wherein the shield 210 is in a first angular position, wherein activation of the drive mechanism is disabled, as the shield structure 210 is not connected to the connector 270. Figures 5B1-5B2 illustrate the drug delivery device in an activation enabled state with the shield 210 in a second angular position. In this state, the shield 210 is arranged to be movable in the axial direction, and thereby allows an axial movement enabling activation of the drive mechanism. In the second angular position the shield structure 210 is connected to the connector 270, which is connected to the drive tube 280. Figure 5C1-5C2 illustrate the drug delivery device 200 in the dosing state, wherein a dose is expelled.

Figure 5A1 illustrate a perspective view of the drug delivery device 200, wherein the tubular elongate housing structure 240 has been removed to illustrate details of the activation and drive mechanism. Figure 5A2 illustrate the same as figure 5A1, but with the difference that the structures are in greyscale to better illustrate the extension and boundary of each structure.

Figure 5A3 illustrate the same parts as figure 5A1, with the exception that an outer tubular ring of the re-lock tube 279 has been removed to illustrate the gap and the not yet established connection between the shield 210 and the connector 270. A portion of an outer tubular portion of the connector has also been removed. Figure 5A4 illustrate the same as 5A3, but with the difference that the structures are in greyscale to better illustrate the extension of each structure.

With reference to figures 5A1-5A4, the shield structure 210 comprises a radial guide 212 arranged at the proximal end and extending radially, whereby the shield is adapted for cooperating with the connector 270. The connector comprises a step-wise helical track 274 for cooperating with the radial guide 212 of the shield. The step-wise helical track comprises a distal transverse portion, a distal helical portion, a middle transverse portion, a proximal helical portion and a proximal transverse portion. Due to the helical portions of the step-wise helical track, a rotational movement of the shield 210 can be transferred into a proximal movement of the shield. The relock-tube is adapted for enabling an automatic re-lock function. The relock function is not relevant for describing the activation and drive mechanism.

As best seen on figure 4A, the connector 270 comprises a first tubular portion 270.1 with a full 360 degree circumference, and a second tubular portion with two cut-outs, whereby the remaining portions of the second tubular portion forms two axially extending tube portions 270.2. The first tubular portion comprises the step-wise helical track 274, and a transverse distally oriented surface of the two axially extending tube portions 270.2 provides the distal guide 272.

As seen on figures 5A1-5A4, the shield can be arranged within the first tubular portion 270.1 with the radial guide extending through the step-wise helical track 274. The axially extending tube portions 270.2 extends in the proximal direction from the inner surface of the first tubular portion 270.1, and therefore has a smaller diameter. The diameter of the tube portions 270.2 corresponds to the diameter of the shield 210, and the proximal guide 214 having a transverse proximally oriented surface is adapted to provide an axial gap to the transverse distally oriented surface of the distal guide 272, when the shield 210 is in the first angular position. The diameter of the axially extending tube portions 270.2 further corresponds to the diameter of middle guide 244 of the housing 240, whereby the axially extending tube portions 270.2 can cooperate with the middle guide 244. The connector 270 is having an outer surface with an outer diameter and an inner surface with an inner diameter. The axial gap between the proximal guide 214 of the shield 210, and a distal guide 272 of the connector 270, is illustrated in figure 5A3.

Figure 5B1-5B2 illustrate the drug delivery device in the activation enabled state, wherein the shield has been rotated and forced to perfom a proximal helical movement, whereby the shield has been positioned in the second angular position. From an illustration point of view, figure 5B1 corresponds to figure 5A1 and figure 5B2 corresponds to figure 5A3. The axial gap between the proximal guide 214 of the shield 210, illustrated in figure 5A3, has been eliminated and a contact has been established between the shield and the connector. As the shield 210 is arranged to be movable in the axial direction, the shield enables axial movement of the connector 270. The connector 270 comprises an activation tab278, which is connected to the drive tube 280, wherein the connector is adapted to move the drive tube 280 in the proximal direction in response to proximal movement of the shield 210.

Although not illustrated, as for the first embodiment of the drug delivery device 100, the drive tube 280 is axially splined to the piston rod 209, whereby relative axial movement between the drive tube and the piston rod is allowed and relative rotational movement is restricted. In the second angular position of the shield 210, the drive tube 280 is adapted to be axially movable relative to the housing assembly between a first axial and non-rotatable position and a second axial and rotatable position, in response to axial movement of the shield 210. The drive tube 280 arranged in the non-rotatable position is rotationally blocked by the housing assembly, and the drive tube 280 arranged in the rotatable position is allowed to rotate and to be guided by the housing assembly.

Although not illustrated, the housing assembly comprises an axial guide portion for blocking rotation of the drive tube 280 in the non-rotatable position, and the housing assembly comprises a helical guide portion for guiding the drive tube 280 in the rotatable position.

Figure 5C1-5C2 illustrate the drug delivery device 200 in the dosing state, wherein the drive tube 280 is in a rotatable position, and a dose is expelled. From an illustration point of view, figure 5C1 corresponds to figure 5B1 and figure 5C2 corresponds to figure 5B2. As best illustrated in figure 5C2, the contact between the proximal guide 214 and the distal guide 272 continues during dosing.

### Safety assembly

The multi-use fixed dose drug delivery device is front-activated by a shield, which retracts when pressure is applied on the shield tip. Alternatively, the device is activated by a push button on the proximal end, which is depressed, when pressure is applied. This shield or push button has a disabled/locked and enabled/unlocked state, requiring the user to grab the shield and turn this to the enabled/unlocked position prior to injection. When the shield or push button is being set into the enabled/unlocked position it is possible for the user to unintentionally press the shield or push button, which will activate dispensing of the dose.

Figs. 6-8 illustrate an embodiment of a safety assembly 300 with the ring member 390. The drug delivery devices 100 and 200 can both be modified to cooperate with the safety assembly 300.

With a ring member attached to the device and rotationally locked to the shield or push button, the user is supposed to set the device in the disabled/locked and enabled/unlocked state by turning the ring member instead of the shield. Thereby minimizing the likelihood of a user unintentionally pressing the shield or push button, and thereby activating the drive mechanism to dispense the drug.

Figure 6 illustrates an exploded view of the safety assembly 300. The safety assembly comprises a ring member 390, a housing insert portion 360 and an elongate shield structure 310. The shield structure 310 comprises a step-wise helical guide corresponding to the step-wise helical guide 112 of the first embodiment. Compared to the step-wise helical guide 112, the middle transverse portion has been removed from the step-wise helical guide 312 for technical reasons relating to the injection moulding process. However, the guide structures 112, 312 functions in the same manner.

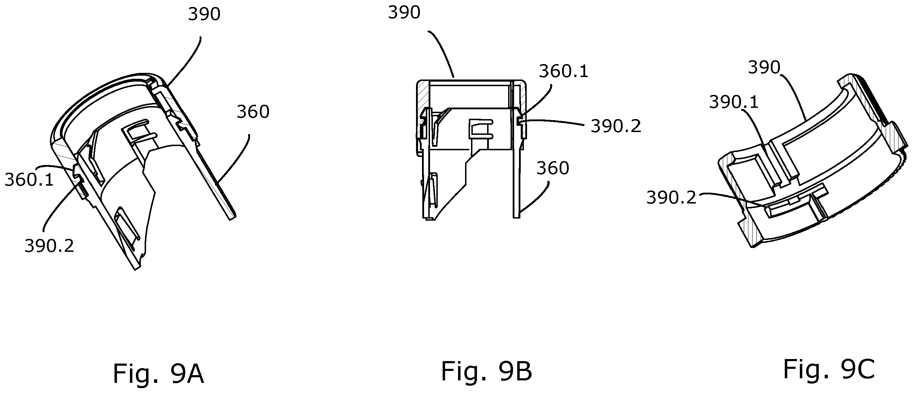

Figure 7 illustrates the safety assembly in an assembled state corresponding to an initial position, wherein the shield 310 is in the distalmost position relative to the housing insert portion 360. The ring member 390 is attached to the housing insert portion 360, and is axially splined to the shield structure 310 via axially extending ribs 316 (figure 6) fitting into axially extending tracks 390.1 (figure 9C). The ring member 390 and the housing insert portion 360 surrounds the shield 310. In the illustrated embodiment two pairs of ribs and tracks are used, but any number of ribs would work as long as a rotation can be transferred from the ring to the shield, and as long the shield can move axially relative to the ring.

Although the spline connection is only illustrated as pairs of ribs and tracks other axially extending structures can also be used, e.g., a corrugated surface. In some embodiments the rib 316 can be substituted with an axially extending structure with a uniform axial cross-section, which is rectangular, triangular or T-shaped. For these embodiments the track 370.1 is substituted with corresponding tracks matching the cross-section of the axially extending structure, wherein the cross-section of these tracks is rectangular, triangular or T-shaped.

If the cross-section is uniform relative axial movement is allowed between the ring and the shield. However, if the track is wider than the corresponding axially extending structure, the cross-section of the axially extending structure may vary slightly in the axial direction. If the track is slightly larger than the axially extending structure, a slight play between the ring and the shield is introduced in the rotational direction.

In general splines are ridges, teeth or corrugations on an inner surface of the ring that mesh with tracks, grooves or ridges in the mating outer surface of the shield. Thereby the ring can transfer torque to the shield, while maintaining the angular correspondence between them. However, a further adaptation to the general principle is that the shield can move relative to the ring. Thereby, the shield can be adapted to activate the drive mechanism, when moved in the axial direction relative to the ring attached to the housing.

When, as an example, the safety assembly 300 is implemented in the first of embodiment 100 of the drug delivery device, the housing insert portion 360 is substituted with the housing insert portion 160, by attaching the insert portion 360 to the elongate housing structure 140. The elongate shield structure 110 is substituted with the elongate shield structure 310 and the surrounding ring member 390.

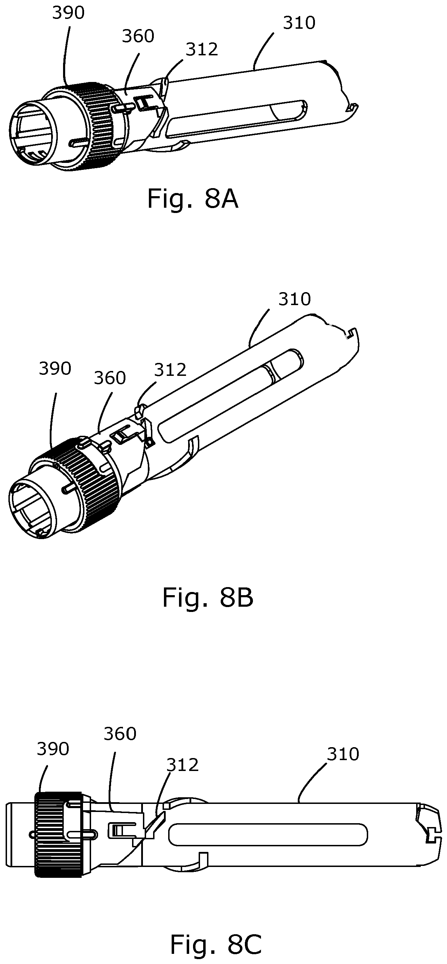

Figure 8A to 8C illustrates the safety assembly device in different angular positions corresponding to different states, when implemented in a drug delivery device. In figure 8A, the shield 310 is in a first angular position corresponding to the cap-off state (C1), wherein the shield 310 is axially lock and wherein the activation of the drive mechanism is disabled. In figure 8C the shield 310 is in a second angular position corresponding to the shield unlocked state (D1), wherein the shield is allowed to move in an axial direction, and wherein activation of the drive mechanism is enabled. Figure 8B illustrates the shield arranged in an angular intermediate position between the first and the second angular position. The figures also illustrates that the step-wise helical guide 312 cooperates with a proximal surface of the housing insert portion 360.

Axially the inner ring is held in place by a snap connection between the ring member 390 and the housing insert portion 360, as shown in figure 9A to 9C. Figures 9A and 9B illustrate in a perspective view the inner surface of the ring member 390 connected to the housing insert portion 360. Figure 9C illustrate details of the inner surface of the ring member 390. A protrusion 360.1 is snapped into a transverse track 390.2.

As also illustrated on figure 9A to 9C, the inner ring comprises an axially extending track 390.1 cooperating with one of the two axially extending ribs 316 on the shield, seen on figure 6, to transfer the rotational motion from the ring member 390 to the shield 310. The drug delivery device with the safety assembly 300 is unlocked by turning the ring member. The shield is then also forced downwards because of the helical contact surfaces between the proximal surface of the housing insert portion 360, and the step-wise helical guide 312. This movement cause the shield to engage the driving mechanism of the device, and activation is enabled.

Similarly, it will be possible to implement a safety assembly in the drug delivery device 200, by modifying the housing cap portion 260 to axially lock a ring member, while the ring member is still allowed to rotate. The ring member should similar be adapted to axially spline with shield 210.

In an alternative embodiment of the drug delivery device, the housing comprises a helical guide 360 for guiding the shield 310 or activation member in the axial direction and into the housing, in response to rotating the shield 310 or activation member from the first angular position to the second angular portion. In a further aspect of the embodiment the shield 310 or activation member comprises a helical guide 312 for engaging the helical guide 360 of the housing during the rotation from the first angular position to the second angular position.

In an alternative embodiment is provided a safety assembly for a drug delivery device for delivering a dose of a medicament, wherein the drug delivery device comprises:
- a housing assembly comprising an elongate housing structure 140, 240,
- a drive mechanism for delivering the dose, the drive mechanism being arranged within the elongate housing structure 140, 210, wherein the drive mechanism is adapted to expel the dose of the medicament, in response to activation,
- an activation mechanism comprising a rotatable tubular activation member 310 adapted to activate the drive mechanism, in response to an axial movement of the activation member, wherein the activation member 310 is adapted to be arranged in: a first angular position, wherein the activation of the drive mechanism is disabled, a second angular position, wherein the activation member 310 is arranged to be movable in the axial direction, and thereby allows an axial movement enabling activation of the drive mechanism,

wherein the safety assembly comprises the activation member 310, a housing insert portion 360 and a ring member 390 surrounding the activation member, wherein the housing insert portion is adapted to be fixed to the housing assembly,
wherein the ring member 390 is connected to the housing insert portion, wherein the ring member 390 is arranged to be axially fixed and movable in the rotational direction relative to the housing insert portion 360, wherein the ring member 390 is axially splined to the activation member 310, whereby the ring member 390 is rotationally locked and movable in the axial direction relative to the activation member 310,
whereby the ring member 390 is adapted to transfer a rotation to the activation member 310 from the first position to the second position, in response to a rotation of the ring member 390, whereby a user can rotate the activation member 310 without unintentionally activating the drive mechanism.

In an alternative embodiment is provided a method of using a drug delivery device according to the previously described embodiments, wherein the method comprises:
- turning the ring member 390 from the first angular position to the second angular position and thereby enabling activation of the drive mechanism, without directly touching the activation member, and thereby not expelling a dose.

In the above description of exemplary embodiments, the different structures and means providing the described functionality for the different components have been described to a degree to which the concept of the present invention will be apparent to the skilled reader. The detailed construction and specification for the different components are considered the object of a normal design procedure performed by the skilled person along the lines set out in the present specification.

## Claims

1. A drug delivery device for delivering a dose of a medicament, wherein the drug delivery device comprises:
- a housing assembly comprising an elongate housing structure (140, 240);
- a drive mechanism for delivering the dose, the drive mechanism being arranged within the elongate housing structure (140, 210), wherein the drive mechanism is adapted to expel the dose of the medicament, in response to activation;
- an activation mechanism comprising a rotatable tubular activation member (310) adapted to activate the drive mechanism, in response to an axial movement of the activation member, wherein the activation member (310) is adapted to be arranged in: (i) a first angular position, wherein the activation of the drive mechanism is disabled, (ii) a second angular position, wherein the activation member (310) is arranged to be movable in the axial direction, and thereby allows an axial movement enabling activation of the drive mechanism;
**characterized in that** the drug delivery device further comprises a ring member (390) surrounding the activation member (310);
wherein the ring member (390) is connected to the housing assembly, wherein the ring member (390) is arranged to be axially fixed and movable in the rotational direction relative to the housing assembly, wherein the ring member (390) is axially splined to the activation member (310), whereby the ring member (390) is rotationally locked and movable in the axial direction relative to the activation member (310);
whereby the ring member (390) is adapted to transfer a rotation to the activation member (310) from the first position to the second position, in response to a rotation of the ring member, whereby a user can rotate the activation member (310) without unintentionally activating the drive mechanism.

2. The drug delivery device according to claim 1, wherein the drug delivery device further comprises:
- a medicament reservoir (135, 235) with an outlet at a distal end (135a),
- a movable piston (136, 236) for compression of the reservoir (135, 235), and
wherein the drive mechanism further comprises a piston rod (109, 209) for moving the piston (136, 236) to compress and expel the medicament through the outlet, an energized power-reservoir operationally arranged for moving the piston rod (108, 208) and for delivering the entire dose.

3. The drug delivery device according to any of claims 1 and 2, wherein the drive mechanism comprises a drive tube (180, 280) axially splined to the piston rod (109, 209), whereby relative axial movement between the drive tube and the piston rod is allowed and relative rotational movement is restricted, wherein the drive tube (180, 280) is adapted to be axially movable relative to the housing assembly between a first axial and non-rotatable position and a second axial and rotatable position, in response to axial movement of the activation member (310), and wherein the drive tube (180, 280) arranged in the non-rotatable position is rotationally blocked by the housing assembly, and wherein the drive tube (180, 280) arranged in the rotatable position is allowed to rotate and guided by the housing assembly.

4. The drug delivery device according to claim 3, wherein the housing assembly comprises an axial guide portion for blocking rotation of the drive tube (180, 280) in the non-rotatable position, and wherein the housing assembly comprises a helical guide portion for guiding the drive tube (180, 280) in the rotatable position.

5. The drug delivery device according to any of claims 3 and 4, wherein the activation mechanism further comprises a connector (170, 270) for establishing a connection between the activation member (110, 210) and the drive tube (180, 280), for the activation member being in the second angular position, whereby the connector (170, 270) can transfer an axial movement of the activation member to the drive tube (180, 280).

6. The drug delivery device according to any of the claims 3-5, wherein the activation member (360) in the first angular position, is connected to the connector (170), and the connector (170) is operationally disconnected from the drive tube (180), whereby activation is disabled, wherein the activation member (360) in the second angular position is connected to the connector (170), and wherein the connector (170) is operationally connected to the drive tube (180), whereby activation is enabled.

7. The drug delivery device according to any of claims 3 to 5, wherein the activation member (310) in the first angular position, is operationally disconnected from the drive tube (280), whereby activation is disabled, wherein the activation member (310) in the second angular position is operationally connected to the drive tube (280), whereby activation is enabled.

8. The drug delivery device according to any of claims 3 to 6, wherein the activation member comprises a blocking structure (112) for blocking against a blocking structure (142) of the housing assembly, wherein the blocking structures (112, 142) are adapted for preventing pure axial movement of the activation member (110) in the first angular position, whereby activation is disabled, wherein the blocking structures (112, 142) are adapted for allowing axial movement of the activation member (110) in the second angular position, whereby activation is enabled.

9. The drug delivery device according to any of claims 3 to 8, wherein the housing assembly further comprises an inner thread, wherein the piston rod (109, 209) further comprises an outer thread engaging the inner thread of the housing.

10. The drug delivery device according to any of claims 3 to 9, wherein the power reservoir comprises a torsion spring (108, 208), wherein the drive tube (180, 280) and the torsion spring (108, 208) are adapted for driving the piston rod (109, 209), in response to activation of the drive tube (180, 280) by the activation member (310).

11. The drug delivery device according to any of claims 1 or 2, wherein the activation member is a proximally positioned push button adapted to be moved axially in the distal direction for activation of the drive mechanism.

12. The drug delivery device according any of the previous claims, wherein the activation member (310) is a shield.

13. The drug delivery device according to any of the previous claims, wherein the drug delivery device is an injection device comprising a needle cannula.

14. The drug delivery device according to any of the previous claims, wherein the drug delivery device is adapted to be a multi-use fixed dose drug delivery device, wherein the activation member is adapted to be operated a plurality of times and thereby moved: (i) from the first angular position to the second angular position to enable activation, (ii) in the axial direction to activate the drive mechanism, (iii) from the second angular position to the first angular position to disable activation.

15. A method of using a drug delivery device according to claim 1, wherein the method comprises:
- turning the ring member (390) from the first angular position to the second angular position and thereby enabling activation of the drive mechanism, without directly touching the activation member, and thereby not expelling a dose.

## Patentansprüche

1. Arzneimittelabgabevorrichtung zur Abgabe einer Dosis eines Medikaments, wobei die Arzneimittelabgabevorrichtung umfasst:
- eine Gehäuseanordnung, die eine längliche Gehäusestruktur (140, 240) umfasst;
- einen Antriebsmechanismus zum Abgeben der Dosis, wobei der Antriebsmechanismus innerhalb der länglichen Gehäusestruktur (140, 210) angeordnet ist, wobei der Antriebsmechanismus dazu ausgelegt ist, die Dosis des Medikaments in Reaktion auf eine Aktivierung auszustoßen;
- einen Aktivierungsmechanismus, der ein drehbares rohrförmiges Aktivierungselement (310) umfasst, das dazu ausgelegt ist, den Antriebsmechanismus in Reaktion auf eine axiale Bewegung des Aktivierungselements zu aktivieren, wobei das Aktivierungselement (310) dazu ausgelegt ist, in einer der folgenden Positionen angeordnet zu sein: (i) einer ersten Winkelposition, in der die Aktivierung des Antriebsmechanismus deaktiviert ist, (ii) einer zweiten Winkelposition, in der das Aktivierungselement (310) so angeordnet ist, dass es in der axialen Richtung beweglich ist, und dadurch eine axiale Bewegung ermöglicht, die die Aktivierung des Antriebsmechanismus ermöglicht;
**dadurch gekennzeichnet, dass** die Arzneimittelabgabevorrichtung ferner ein Ringelement (390) umfasst, das das Aktivierungselement (310) umgibt;
wobei das Ringelement (390) mit der Gehäuseanordnung verbunden ist, wobei das Ringelement (390) so angeordnet ist, dass es axial fest und in der Drehrichtung relativ zu der Gehäuseanordnung beweglich ist, wobei das Ringelement (390) axial mit dem Aktivierungselement (310) verzahnt ist, wodurch das Ringelement (390) drehfest und in der axialen Richtung relativ zu dem Aktivierungselement (310) beweglich ist;
wobei das Ringelement (390) dazu ausgelegt ist, in Reaktion auf eine Drehung des Ringelements eine Drehung von der ersten Position in die zweite Position auf das Aktivierungselement (310) zu übertragen, wodurch ein Benutzer das Aktivierungselement (310) drehen kann, ohne den Antriebsmechanismus unbeabsichtigt zu aktivieren.

2. Arzneimittelabgabevorrichtung nach Anspruch 1, wobei die Arzneimittelabgabevorrichtung ferner umfasst:
- einen Medikamentenbehälter (135, 235) mit einem Auslass an einem distalen Ende (135a),
- einen beweglichen Kolben (136, 236) zum Komprimieren des Behälters (135, 235), und wobei der Antriebsmechanismus ferner eine Kolbenstange (109, 209) zum Bewegen des Kolbens (136, 236) zum Komprimieren und Ausstoßen des Medikaments durch den Auslass umfasst, wobei ein energetisierter Kraftspeicher betriebsmäßig zum Bewegen der Kolbenstange (108, 208) und zum Abgeben der gesamten Dosis angeordnet ist.

3. Arzneimittelabgabevorrichtung nach einem der Ansprüche 1 und 2, wobei der Antriebsmechanismus ein Antriebsrohr (180, 280) umfasst, das axial mit der Kolbenstange (109, 209) verzahnt ist, wodurch eine relative axiale Bewegung zwischen dem Antriebsrohr und der Kolbenstange ermöglicht und eine relative Drehbewegung eingeschränkt wird, wobei das Antriebsrohr (180, 280) in Reaktion auf eine axiale Bewegung des Aktivierungselements (310) axial relativ zu der Gehäuseanordnung zwischen einer ersten axialen und nicht drehbaren Position und einer zweiten axialen und drehbaren Position bewegbar ist und wobei das Antriebsrohr (180, 280), in der nicht drehbaren Position angeordnet, durch die Gehäuseanordnung drehfest blockiert ist, und wobei sich das Antriebsrohr (180, 280), in der drehbaren Position, drehen kann und durch die Gehäuseanordnung geführt wird.

4. Arzneimittelabgabevorrichtung nach Anspruch 3, wobei die Gehäuseanordnung einen axialen Führungsabschnitt zum Blockieren der Drehung des Antriebsrohrs (180, 280) in der nicht drehbaren Position umfasst, und wobei die Gehäuseanordnung einen schraubenförmigen Führungsabschnitt zum Führen des Antriebsrohrs (180, 280) in der drehbaren Position umfasst.

5. Arzneimittelabgabevorrichtung nach einem der Ansprüche 3 und 4, wobei der Aktivierungsmechanismus ferner ein Verbindungselement (170, 270) zum Herstellen einer Verbindung zwischen dem Aktivierungselement (110, 210) und dem Antriebsrohr (180, 280) umfasst, wobei sich das Aktivierungselement in der zweiten Winkelposition befindet, wodurch das Verbindungselement (170, 270) eine axiale Bewegung des Aktivierungselements auf das Antriebsrohr (180, 280) übertragen kann.

6. Arzneimittelabgabevorrichtung nach einem der Ansprüche 3-5, wobei das Aktivierungselement (360) in der ersten Winkelposition mit dem Verbindungselement (170) verbunden ist und das Verbindungselement (170) betriebsmäßig von dem Antriebsrohr (180) getrennt ist, wodurch die Aktivierung deaktiviert ist, wobei das Aktivierungselement (360) in der zweiten Winkelposition mit dem Verbindungselement (170) verbunden ist, und wobei das Verbindungselement (170) betriebsmäßig mit dem Antriebsrohr (180) verbunden ist, wodurch die Aktivierung aktiviert ist.

7. Arzneimittelabgabevorrichtung nach einem der Ansprüche 3 bis 5, wobei das Aktivierungselement (310) in der ersten Winkelposition betriebsmäßig von dem Antriebsrohr (280) getrennt ist, wodurch die Aktivierung deaktiviert ist, wobei das Aktivierungselement (310) in der zweiten Winkelposition betriebsmäßig mit dem Antriebsrohr (280) verbunden ist, wodurch die Aktivierung aktiviert ist.

8. Arzneimittelabgabevorrichtung nach einem der Ansprüche 3 bis 6, wobei das Aktivierungselement eine Sperrstruktur (112) zum Blockieren gegen eine Sperrstruktur (142) der Gehäuseanordnung umfasst, wobei die Sperrstrukturen (112, 142) dazu ausgelegt sind, eine rein axiale Bewegung des Aktivierungselements (110) in der ersten Winkelposition zu verhindern, wodurch die Aktivierung deaktiviert ist, wobei die Sperrstrukturen (112, 142) dazu ausgelegt sind, eine axiale Bewegung des Aktivierungselements (110) in der zweiten Winkelposition zu ermöglichen, wobei die Aktivierung aktiviert ist.

9. Arzneimittelabgabevorrichtung nach einem der Ansprüche 3 bis 8, wobei die Gehäuseanordnung ferner ein Innengewinde umfasst, wobei die Kolbenstange (109, 209) ferner ein Außengewinde umfasst, das mit dem Innengewinde des Gehäuses in Eingriff steht.

10. Arzneimittelabgabevorrichtung nach einem der Ansprüche 3 bis 9, wobei der Kraftspeicher eine Torsionsfeder (108, 208) umfasst, wobei das Antriebsrohr (180, 280) und die Torsionsfeder (108, 208) dazu ausgelegt sind, die Kolbenstange (109, 209) in Reaktion auf eine Aktivierung des Antriebsrohrs (180, 280) durch das Aktivierungselement (310) anzutreiben.

11. Arzneimittelabgabevorrichtung nach einem der Ansprüche 1 oder 2, wobei das Aktivierungselement ein proximal positionierter Druckknopf ist, der so ausgelegt ist, dass er zur Aktivierung des Antriebsmechanismus axial in distaler Richtung bewegt werden kann.

12. Arzneimittelabgabevorrichtung nach einem der vorhergehenden Ansprüche, wobei das Aktivierungselement (310) ein Schild ist.

13. Arzneimittelabgabevorrichtung nach einem der vorhergehenden Ansprüche, wobei die Arzneimittelabgabevorrichtung eine Injektionsvorrichtung ist, die eine Nadelkanüle umfasst.

14. Arzneimittelabgabevorrichtung nach einem der vorhergehenden Ansprüche, wobei die Arzneimittelabgabevorrichtung dazu ausgelegt ist, eine wiederverwendbare Arzneimittelabgabevorrichtung mit fester Dosis zu sein, wobei das Aktivierungselement dazu ausgelegt ist, mehrfach betätigt und dadurch bewegt zu werden: (i) von der ersten Winkelposition zu der zweiten Winkelposition, um eine Aktivierung zu ermöglichen, (ii) in der axialen Richtung, um den Antriebsmechanismus zu aktivieren, (iii) von der zweiten Winkelposition zu der ersten Winkelposition, um die Aktivierung zu deaktivieren.

15. Verfahren zur Verwendung einer Arzneimittelabgabevorrichtung nach Anspruch 1, wobei das Verfahren umfasst:
- Drehen des Ringelements (390) von der ersten Winkelposition zu der zweiten Winkelposition und dadurch Ermöglichen der Aktivierung des Antriebsmechanismus, ohne das Aktivierungselement direkt zu berühren, wodurch keine Dosis ausgestoßen wird.

## Revendications

1. Dispositif d'administration de médicament pour administrer une dose d'un médicament, dans lequel le dispositif d'administration de médicament comprend :
- un ensemble logement comprenant une structure de logement allongée (140, 240) ;
- un mécanisme d'entraînement pour administrer la dose, le mécanisme d'entraînement étant agencé au sein de la structure de logement allongée (140, 210), dans lequel le mécanisme d'entraînement est adapté pour expulser la dose du médicament, en réponse à l'activation ;
- un mécanisme d'activation comprenant un élément d'activation tubulaire rotatif (310) adapté pour activer le mécanisme d'entraînement, en réponse à un mouvement axial de l'élément d'activation, dans lequel l'élément d'activation (310) est adapté pour être agencé dans : (i) une première position angulaire, dans lequel l'activation du mécanisme d'entraînement est désactivée, (ii) une deuxième position angulaire, dans lequel l'élément d'activation (310) est agencé pour être mobile dans la direction axiale, et autorise ainsi un mouvement axial permettant l'activation du mécanisme d'entraînement ;
**caractérisé en ce que** le dispositif d'administration de médicament comprend en outre un élément annulaire (390) entourant l'élément d'activation (310) ;
dans lequel l'élément annulaire (390) est raccordé à l'ensemble logement, dans lequel l'élément annulaire (390) est agencé pour être axialement fixe et mobile dans la direction de rotation par rapport à l'ensemble logement, dans lequel l'élément annulaire (390) est axialement cannelé par rapport à l'élément d'activation (310), moyennant quoi l'élément annulaire (390) est bloqué en rotation et mobile dans la direction axiale par rapport à l'élément d'activation (310) ;
moyennant quoi l'élément annulaire (390) est adapté pour transférer une rotation à l'élément d'activation (310) de la première position à la deuxième position, en réponse à une rotation de l'élément annulaire, moyennant quoi un utilisateur peut faire tourner l'élément d'activation (310) sans activer de manière non intentionnelle le mécanisme d'entraînement.

2. Dispositif d'administration de médicament selon la revendication 1, dans lequel le dispositif d'administration de médicament comprend en outre :
- un réservoir de médicament (135, 235) avec une sortie au niveau d'une extrémité distale (135a),
- un piston mobile (136, 236) pour la compression du réservoir (135, 235), et
dans lequel le mécanisme d'entraînement comprend en outre une tige de piston (109, 209) pour déplacer le piston (136, 236) afin de comprimer et d'expulser le médicament à travers la sortie, un réservoir d'énergie alimenté fonctionnellement étant agencé pour déplacer la tige de piston (108, 208) et pour administrer la dose entière.

3. Dispositif d'administration de médicament selon l'une quelconque des revendications 1 et 2, dans lequel le mécanisme d'entraînement comprend un tube d'entraînement (180, 280) cannelé axialement par rapport à la tige de piston (109, 209), moyennant quoi un mouvement axial relatif entre le tube d'entraînement et la tige de piston est autorisé et un mouvement de rotation relatif est restreint, dans lequel le tube d'entraînement (180, 280) est adapté pour être mobile axialement par rapport à l'ensemble logement entre une première position axiale et non rotative et une deuxième position axiale et rotative, en réponse au mouvement axial de l'élément d'activation (310), et dans lequel le tube d'entraînement (180, 280) agencé dans la position non rotative est bloqué en rotation par l'ensemble logement, et dans lequel le tube d'entraînement (180, 280) agencé dans la position rotative est autorisé à tourner et à être guidé par l'ensemble logement.

4. Dispositif d'administration de médicament selon la revendication 3, dans lequel l'ensemble logement comprend une portion de guidage axiale pour bloquer la rotation du tube d'entraînement (180, 280) dans la position non rotative, et dans lequel l'ensemble logement comprend une portion de guidage hélicoïdale pour guider le tube d'entraînement (180, 280) dans la position rotative.

5. Dispositif d'administration de médicament selon l'une quelconque des revendications 3 et 4, dans lequel le mécanisme d'activation comprend en outre un raccord (170, 270) pour établir un raccordement entre l'élément d'activation (110, 210) et le tube d'entraînement (180, 280), l'élément d'activation étant dans la deuxième position angulaire, moyennant quoi le raccord (170, 270) peut transférer un mouvement axial de l'élément d'activation au tube d'entraînement (180, 280).

6. Dispositif d'administration de médicament selon l'une quelconque des revendications 3 à 5, dans lequel l'élément d'activation (360) dans la première position angulaire, est raccordé au raccord (170), et le raccord (170) est fonctionnellement ôté du raccordement au tube d'entraînement (180), moyennant quoi l'activation n'est plus permise, dans lequel l'élément d'activation (360) dans la deuxième position angulaire est raccordé au raccord (170), et dans lequel le raccord (170) est fonctionnellement raccordé au tube d'entraînement (180), moyennement quoi l'activation est permise.

7. Dispositif d'administration de médicament selon l'une quelconque des revendications 3 à 5, dans lequel l'élément d'activation (310) dans la première position angulaire, est fonctionnellement ôté du raccordement au tube d'entraînement (280), moyennant quoi l'activation est n'est plus permise, dans lequel l'élément d'activation (310) dans la deuxième position angulaire est fonctionnellement raccordé au tube d'entraînement (280), moyennant quoi l'activation est permise.

8. Dispositif d'administration de médicament selon l'une quelconque des revendications 3 à 6, dans lequel l'élément d'activation comprend une structure de blocage (112) pour bloquer contre une structure de blocage (142) de l'ensemble logement, dans lequel les structures de blocage (112, 142) sont adaptées pour empêcher un mouvement axial pur de l'élément d'activation (110) dans la première position angulaire, moyennant quoi l'activation n'est plus permise, dans lequel les structures de blocage (112, 142) sont adaptées pour autoriser un mouvement axial de l'élément d'activation (110) dans la deuxième position angulaire, dans lequel l'activation est permise.

9. Dispositif d'administration de médicament selon l'une quelconque des revendications 3 à 8, dans lequel l'ensemble logement comprend en outre un filetage intérieur, dans lequel la tige de piston (109, 209) comprend en outre un filetage extérieur venant en prise avec le filetage intérieur du logement.

10. Dispositif d'administration de médicament selon l'une quelconque des revendications 3 à 9, dans lequel le réservoir de puissance comprend un ressort de torsion (108, 208), dans lequel le tube d'entraînement (180, 280) et le ressort de torsion (108, 208) sont adaptés pour entraîner la tige de piston (109, 209), en réponse à l'activation du tube d'entraînement (180, 280) par l'élément d'activation (310).

11. Dispositif d'administration de médicament selon l'une quelconque des revendications 1 ou 2, dans lequel l'élément d'activation est un bouton poussoir positionné de manière proximale adapté pour être déplacé axialement dans la direction distale pour l'activation du mécanisme d'entraînement.

12. Dispositif d'administration de médicament selon l'une quelconque des revendications précédentes, dans lequel l'élément d'activation (310) est une protection.

13. Dispositif d'administration de médicament selon l'une quelconque des revendications précédentes, dans lequel le dispositif d'administration de médicament est un dispositif d'injection comprenant une canule d'aiguille.

14. Dispositif d'administration de médicament selon l'une quelconque des revendications précédentes, dans lequel le dispositif d'administration de médicament est adapté pour être un dispositif d'administration de médicament à dose fixe et à usage multiple, dans lequel l'élément d'activation est adapté pour être mis en fonctionnement une pluralité de fois et ainsi déplacé : (i) de la première position angulaire à la deuxième position angulaire pour permettre l'activation, (ii) dans la direction axiale pour activer le mécanisme d'entraînement, (iii) de la deuxième position angulaire à la première position angulaire pour ne plus permettre l'activation.

15. Procédé d'utilisation d'un dispositif d'administration de médicament selon la revendication 1, dans lequel le procédé comprend :
- le fait de tourner l'élément annulaire (390) de la première position angulaire à la deuxième position angulaire et ainsi permettre l'activation du mécanisme d'entraînement, sans toucher directement l'élément d'activation, et ainsi ne pas expulser une dose.
